# EUROPÄISCHE PATENTANMELDUNG

(11) **EP 1 854 425 A1**
(43) Veröffentlichungstag der Anmeldung: **14.11.2007**
(21) Anmeldenummer: 06009743.3
(22) Anmeldetag: 11.05.2006
(51) Int. Cl.: A61B 19/00, B25J 9/16

(54) **Medizintechnische Positionsbestimmung mit redundanten Positionserfassungseinrichtungen und Prioritätsgewichtung für die Positionserfassungseinrichtungen**

(71) Anmelder: BrainLAB AG, 85551 Kirchheim/Heimstetten (DE)
(72) Erfinder: Wohlgemuth, Richard, 83646 Bad Tölz (DE)
(74) Vertreter: Rögner, Jürgen

(57) **Zusammenfassung**

Die Erfindung betrifft ein Verfahren zur Bestimmung der Position einer medizintechnischen Einrichtung oder eines Patientenkörperteils, bei dem
- mindestens zwei Positionserfassungen, insbesondere eine erste und eine zweite Positionserfassung, für die Einrichtung oder den Körperteil mit separaten Positionserfassungseinrichtungen durchgeführt werden;
- auf der Basis mindestens einer definierten Eingangsgröße den jeweiligen Positionserfassungen eine bestimmte Priorität zugeordnet wird, welche die Gewichtung der jeweiligen Positionserfassungen bei der Positionsbestimmung definiert; und bei dem
- die Positionsbestimmung unter Berücksichtigung der Gewichtung der jeweiligen Positionserfassungen aus deren Kombination erfolgt.

Sie betrifft auch ein Verfahren zum Steuern eines medizintechnischen Manipulators, insbesondere eines medizintechnischen Roboters, bei dem der Manipulator mit Hilfe von Positionsinformationen gesteuert wird, die mit einem solchen Positionsbestimmungsverfahren erhalten wurden, sowie entsprechende Positionsbestimmungs- und Steuerungsvorrichtungen.

## Beschreibung

Die Erfindung betrifft die medizintechnische Positionsbestimmung mit redundanten Positionserfassungseinrichtungen und Prioritätsgewichtung für die Positionserfassungseinrichtungen. In jüngerer Zeit werden Operationsräume immer häufiger mit so genannten Navigationssystemen ausgestattet. Diese Navigationssysteme helfen behandelnden Ärzten dabei, die von ihnen verwendeten Behandlungseinrichtungen (Instrumente) im Positionsverhältnis zu Patienten oder Patientenkörperteilen zu bestimmen, und sie liefern behandlungsunterstützende Bildausgaben. Um eine funktionierende Navigation bereitstellen zu können, ist es notwendig, die Positionen der Behandlungseinrichtungen bzw. der behandlungsunterstützenden Einrichtungen sowie des Patienten in einem vorgegebenen Koordinatensystem zu bestimmen und zu verfolgen. Diesen Vorgang nennt man "Tracking". Es gibt optische oder magnetische Trackingsysteme, welche Referenzmarker oder Referenzmarkeranordnungen orten und verfolgen, die an den Behandlungseinrichtungen oder am Patienten angebracht sind. Es ist aber beispielsweise auch möglich, ein getracktes Instrument zu verwenden, um andere Objekte oder Patiententeile anzufahren und deren Lage auf diese Weise mit Hilfe des Trackingsystems zu identifizieren. Eine andere Art von Trackingsystem ist speziell für Manipulatoren, wie zum Beispiel medizintechnische oder chirurgische Roboter, anwendbar, wobei in Gelenken dieser Manipulatoren Sensoren eingebracht sind, welche die Bewegung eines Instruments an der Manipulatorspitze gegenüber einer vorkalibrierten Nullstellung ermitteln können.

Es ist auch schon bekannt, solche Positionserfassungseinrichtungen zur redundanten Positionsmessung einzusetzen. Beispielsweise werden dabei "externe Daten", die über ein Kameratrackingsystem für anzubringende Referenzmarkeranordnungen erhalten werden, in ein Roboterkoordinatensystem übertragen und als Eingabedaten für die Robotersteuerung verwendet. Der Roboter, der ein "internes Trackingsystem", also beispielsweise ein Gelenksensoren-Trackingsystem aufweist, erfasst eigene Trackingdaten, und die Redundanz der Gelenksensordaten und der Daten des externen Trackingsystems wird verwendet, um einen Ausfall einer Trackingdatenquelle zu kompensieren, so dass die Behandlung bzw. die Behandlungsunterstützung nicht unterbrochen werden muss. Wenn die Daten der redundanten Positionserfassungen nicht übereinstimmen, wird die Behandlungsunterstützung bzw. der Roboterbetrieb angehalten, die Bewegung eines Gelenkarmes wird gestoppt oder der Betrieb eines aktiven Werkzeugs wird eingestellt.

Aus der US 6,547,782 B1 ist ein Verfahren bekannt, welches die Bewegung eines Instrumentes, das an einem Roboter angebracht ist, innerhalb eines Patienten auf einem vorbestimmten Plan beschränkt. Die Verwendung redundanter Gelenksensoren wird offenbart.

Die EP 0 456 103 A3 bzw. die US 5,086,401 offenbaren ein Verfahren zur redundanten Konsistenzprüfung eines chirurgischen Roboters mit der Positionskontrolle eines Werkzeugs und eine Sicherheitsüberwachung der Werkzeugposition durch ein Kamerasystem. Der Betriebszustand wird periodisch überwacht und es werden Einrichtungen bereitgestellt, um eine weitere Bewegung des Werkzeugs zu verhindern, wenn der Betriebszustand nicht optimal ist.

Redundante Positionserfassungen werden also bisher immer dazu verwendet, die Sicherheit automatischer oder geführter Behandlungsabläufe zu verbessern, indem bei unterschiedlichen erfassten Positionen die Behandlung angehalten wird oder eine Positionsdatenquelle die andere ersetzt, wenn letztere ausfällt.

Es ist die Aufgabe der vorliegenden Erfindung, eine medizintechnische Positionsbestimmung mit redundanten Positionserfassungseinrichtungen vorzuschlagen, bei der die Informationen aus den jeweiligen Positionserfassungen optimal genutzt werden. Diese Aufgabe wird erfindungsgemäß durch ein Positionsbestimmungsverfahren gemäß dem Anspruch 1 sowie durch eine Positionsbestimmungsvorrichtung gemäß dem Anspruch 18 gelöst. Die Unteransprüche definieren bevorzugte Ausfiihrungsformen der Erfindung.

Erfindungsgemäß wird ein Verfahren zur Bestimmung der Position einer medizinischen Einrichtung oder eines Patientenkörperteils beschrieben, bei dem
- mindestens zwei Positionserfassungen, insbesondere eine erste und eine zweite Positionserfassung, für die Einrichtung oder den Körperteil mit separaten Positionserfassungseinrichtungen durchgefüzhrt werden;
- auf der Basis mindestens einer definierten Eingangsgröße den jeweiligen Positionserfassungen eine bestimmte Priorität zugeordnet wird, welche die Gewichtung der jeweiligen Positionserfassungen bei der Positionsbestimmung definiert; und bei dem
- die Positionsbestimmung unter Berücksichtigung der Gewichtung der jeweiligen Positionserfassungen aus deren Kombination erfolgt.

Es werden also bei der vorliegenden Erfindung nicht einfach die erhaltenen Positionsdaten aus redundanten Systemen so "hingenommen" wie sie erhalten werden, sondern die Positionsinformationen werden bewertet. Redundanten Positionsdaten wird eine Priorität zugeordnet, um so die Gewichtung der Positionsdaten basierend auf der Priorität zu bestimmen und eine Position auf der Basis der gewichteten redundanten Positionen zu berechnen, und diese berechnete Position kann dann als Eingabedateninformation für eine medizintechnische Behandlung verwendet werden. Die Erfindung erhöht damit die Sicherheit und Genauigkeit der navigationsgestützten Behandlung, weil sie erstmals in Betracht zieht, dass die Verlässlichkeit und Genauigkeit von Positionsdaten sich während einer Behandlung abhängig von äußeren Einflüssen ändern können. Weil diese äußeren Einflüsse unterschiedliche Wirkungen auf die Verlässlichkeit und Genauigkeit unterschiedlicher Trackingeinrichtungen haben können, optimiert das erfindungsgemäß vorgeschlagene Verfahren gerade die Verlässlichkeit und Genauigkeit über den gesamten Behandlungszeitraum.

Außerdem wird die Nutzung der von verschiedenen Positionserfassungseinrichtungen, die von gleicher oder unterschiedlicher Art sein können, erhaltenen Daten insgesamt optimiert, da im Gegensatz zum Stand der Technik nicht einfach standardmäßig einer Datenquelle der Vorzug gegeben wird und die Informationen der anderen Datenquelle vernachlässigt werden. Durch die Gewichtung und Priorisierung lassen sich Positionsinformationen durchaus auch aus mehreren Erfassungseinrichtungen verwenden, auch wenn diese unterschiedlich sein sollten.

Vorzugsweise erfolgen die jeweiligen Positionserfassungen für einen Zeitpunkt, insbesondere für denselben Zeitpunkt innerhalb des Behandlungsverlaufs. Die Gewichtung der jeweiligen Positionserfassungen kann größer als 0% und kleiner als 100% sein.

Die genannte(n) Eingangsgröße(n) beschreibt bzw. beschreiben insbesondere die Verlässlichkeit und/oder die Genauigkeit der jeweiligen Positionserfassung, und die Eingangsgrößen können externe Parameter und/oder vordefinierte Prioritätsregeln umfassen. Die Erfindung umfasst Ausfiihrungsformen des Verfahrens, bei denen die Eingangsgröße(n) mindestens eine der folgenden Größen umfasst bzw. umfassen:
- den gemessenen Abstand zwischen einer medizintechnischen Einrichtung und einem Körperteil;
- den Zeitabstand zur letzten Kalibrierung einer jeweiligen Positionserfassungseinrichtung;
- die momentane Erfassbarkeit einer jeweiligen Positionserfassungseinrichtung;
- den Unterschied zwischen den von den jeweiligen Positionserfassungseinrichtungen erfassten Positionen;
- die Berechnung des Verhältnisses der Priorität einer Positionserfassungseinrichtung zu einer anderen Positionserfassungseinrichtung deren Positionserfassung konstant ist;
- einen Durchschnitts- oder Zwischenwert der erfassten Positionen.

Wenn der gemessene Abstand zwischen einer medizintechnischen Einrichtung und einem Körperteil als Eingangsgröße verwendet wird, kann für jeden Abstand oder jeden Abstandsbereich eine spezielle Priorität zugeordnet werden. Wenn der Zeitabstand zur letzten Kalibrierung einer jeweiligen Positionserfassungseinrichtung als Eingangsgröße verwendet wird, kann die Referenzposition durch eine der Positionserfassungseinrichtungen bereitgestellt werden, beispielsweise indem die Einrichtung bzw. der Körperteil, der positionsmäßig bestimmt werden soll, mit dem Funktionselement der gewählten Positionserfassungseinrichtung angefahren wird (also zum Beispiel mit der Spitze eines getrackten Instruments unter Kalibrierung kann u.a. auch verstanden werden, dass einer ersten Positionserfassungseinrichtung, welche die Position eines Patienten nicht kennt (z.B. Gelenkroboter) initial die relative Lage des Patienten zu der ersten Positionserfassungseinrichtung durch eine zweite Positionserfassungseinrichtung übermittelt wird.

Bei der Positionsbestimmung können, insbesondere auf Abfrage, noch weitere Regelgrößen berücksichtigt werden, speziell Benutzereingaben, Benutzerbestätigungen oder Eingriffe in die Positionsbestimmung durch einen Benutzer, insbesondere Benutzer-Override-Eingaben.

Es ist erfindungsgemäß möglich, die Priorität einer Positionserfassung auf einen bestimmten Zeitabschnitt einzugrenzen. Die Prioritätszuordnungen können im System aufgezeichnet werden, damit man später auf sie zurückgreifen kann.

Bei einer speziellen Ausführungsform betrifft die Erfindung ein Verfahren, wie es oben beschrieben wurde, bei dem eine der Positionserfassungseinrichtungen ein medizintechnischer Manipulator, insbesondere ein medizintechnischer oder chirurgischer Gelenkroboter ist und eine weitere Positionserfassungseinrichtung ein medizintechnisches optisches oder magnetisches Trackingsystem ist, wobei insbesondere die Eingangsgröße(n) mindestens eine der folgenden Größen umfasst bzw. umfassen:
- die Anzahl der vom Trackingsystem erfassten Referenzmarkierungen, speziell die Anzahl von erfassten Markierungen innerhalb einer Referenzmarkierungsgruppe oder -anordnung bzw. die Qualität der Erfassung;
- die Differenz zwischen Positionsdaten von mindestens zwei redundanten Gelenkpositionssensoren des Roboters;
- Kräfte, die von Kraftsensoren am Roboter gemessen werden;
- die Differenz zwischen Positionsdaten vom Gelenkroboter und vom Trackingsystem;
- die Position der Tracking-Referenzmarkierungen im Sichtfeld des Trackingsystems;
- bei durch das Trackingsystem getrackter Roboterbasis, der Abstand bzw. die zeitliche Änderung des Abstandes zwischen der Basis und der medizintechnischen Einrichtung / dem Körperteil;

Zwischen mehreren Robotern können redundante Positionsdaten und Prioritätszuordnungen ausgetauscht werden. Die Bestimmung der Priorität der Positionsdaten für einen Robtor kann dann in Abhängigkeit von den Prioritätseinstellungen mindestens eines anderen Roboters erfolgen.

Die Erfindung betrifft auch ein Verfahren zum Steuern eines medizinischen Manipulators, insbesondere eines medizintechnischen (chirurgischen) Roboters, bei dem der Manipulator mit Hilfe von Positionsinformationen gesteuert wird, die mit einem Positionsbestimmungsverfahren erhalten wurden, wie es oben in verschiedenen Ausführungen beschrieben wurde. Die Positionsinformationen aus dem Positionsbestimmungsverfahren dienen dann als Eingabedaten für den Manipulator, damit dieser das Behandlungsziel optimal anfährt, und es können dabei die Positionsdaten mit der höchsten Priorität oder aus den redundanten Daten errechnete, verbesserte Positionsinformationen verwendet werden. Im speziellen Fall sind die verwendeten Positionsdaten also eine bestimmte Funktion der redundanten Positionsdaten und ihrer zugeordneten Priorität. Die Positionseingabedaten für die Manipulator- bzw. Robotersteuerung können geändert werden, wenn eine Änderung in der Priorität erfolgt; sie können auch durch einen Benutzereingriff geändert werden. Bei einer speziellen Ausführung werden die Positionseingabedaten nur dann geändert, wenn der Unterschied zwischen verschiedenen Positionserfassungsdaten einen Schwellwert erreicht oder überschreitet.

Es besteht die Möglichkeit, mittels einer Signalausgabe die Gewichtung der Positionserfassungen für den Benutzer anzuzeigen; dies kann optisch oder akustisch, bespielsweise durch Bildschirmanzeigen oder Anzeigelichter einerseits oder durch unterschiedliche Audioausgaben andererseits geschehen.

Die Erfindung umfasst weiter ein Programm, das, wenn es auf einem Computer läuft oder in einem Computer geladen ist, den Computer veranlasst, ein Verfahren durchzufiihren, wie es oben in verschiedenen Ausführungen beschrieben wurde, sowie ein Computerprogramm-Speichermedium, das ein solches Programm aufweist.

Eine erfindungsgemäße Vorrichtung zur Bestimmung der Position einer medizintechnischen Einrichtung oder eines Patientenkörperteils hat mindestens zwei separate Positionserfassungseinrichtungen zur Positionserfassung für die Einrichtung oder den Körperteil, und sie weist eine Steuerungs- und Datenverarbeitungseinheit auf, die eine Speicher- oder Eingabeeinheit für mindestens eine definierte Eingangsgröße umfasst, und welche auf der Basis der mindestens einen definierten Eingangsgröße den jeweiligen Positionserfassungen eine bestimmte Priorität zuordnet, welche die Gewichtung der jeweiligen Positionserfassungen bei der Positionsbestimmung definiert, wobei durch die Steuerungs- und Datenverarbeitungseinheit dann die Position der Einrichtung oder des Körperteils unter Berücksichtung der Gewichtung der jeweiligen Positionserfassungen aus deren Kombination bestimmt wird.

Eine erfindungsgemäße Steuerungsvorrichtung für einen medizintechnischen Manipulator, insbesondere einen medizintechnischen Roboter (chirurgischen Roboter) der mit Hilfe von Positionsinformationen gesteuert wird, ist mit einer Positionsbestimmungsvorrichtung versehen, wie sie oben beschrieben wurde. Natürlich können die erfindungsgemäße Positionsbestimmungsvorrichtung und die erfindungsgemäße Steuerungsvorrichtung all diejenigen Bauteile und Elemente enthalten, die oben in Bezug auf das erfindungsgemäße Verfahren näher beschrieben wurden und die entsprechenden Funktionen erfüllen.

Die Erfindung wird im Weiteren anhand einer Ausführungsform näher erläutert. Sie kann alle hierin beschriebenen Merkmale einzeln bzw. in jeder sinnvollen Kombination umfassen. In den Zeichnungen zeigen:
- Figur 1: ein schematisch dargestelltes, redundantes Tracking-Setup gemäß der Erfindung; und
- Figur 2: ein Ablaufdiagramm für eine erfindungsgemäße Positionsbestimmung und Robotersteuerung.

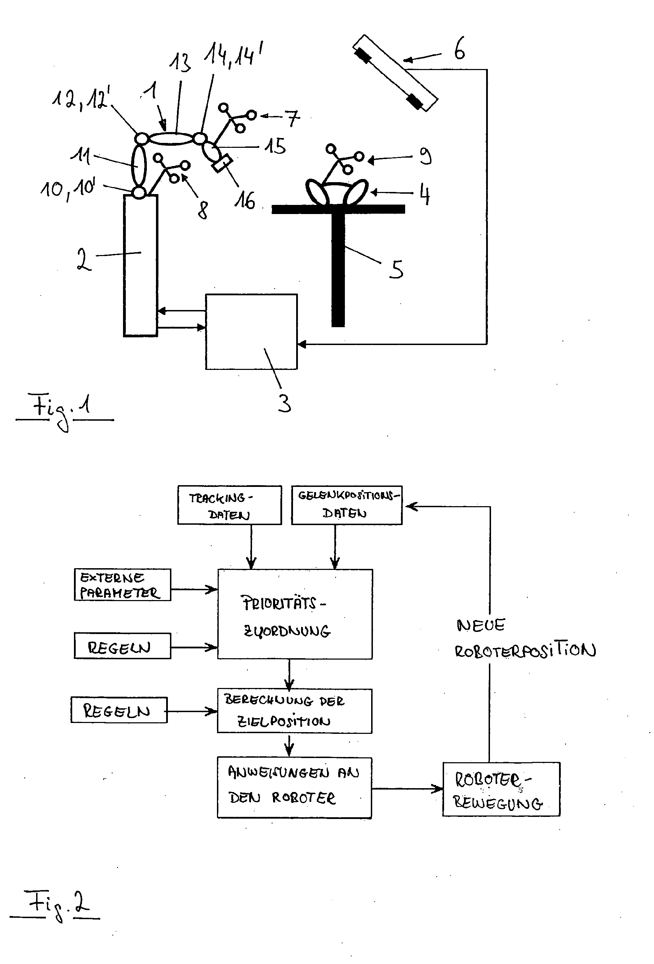

In der Figur 1 ist ein Setup für einen medizinischen Behandlungsraum zu sehen, in dem ein chirurgischer Roboter 1 auf seiner Basis 2 aufgestellt ist. Der Roboter 1 weist ausgehend von seiner Basis ein erstes Gelenk 10 mit Gelenkpositionssensor 10' auf, an das sich ein erstes Armteil 11 anschließt, auf welches wiederum ein Gelenk 12 mit Gelenkpositionssensor 12' folgt. Am Gelenk 12 hängt das zweite Armteil 13 und danach folgt ein drittes Gelenk 14 mit Gelenksensor 14', das wiederum das dritte Armteil 15 trägt, an dem vorne schematisch das Funktionsbauteil 16 dargestellt ist, das durch den Roboter 1 bedient wird. Es können Roboter mit mindestens einem, aber auch mehr als 3 Gelenken verwendet werden (z.B. siehe Gelenke). Die Basis 2 des Roboters 1 ist mit einer Referenzanordnung 8 mit drei Referenzmarkern versehen, ebenso ist das dritte Armteil 15, das gegenüber dem Funktionselement 16 nicht beweglich ist, mit einer entsprechenden Referenzanordnung 7 ausgestattet. Die Referenzanordnung 7 kann auch direkt an einem Instrument angeordnet sein, das vom Roboter (oder von dem Funktionselement) gehalten wird.

Ferner ist in der Figur 1 noch ein Patiententisch 5 gezeigt, auf dem ein Patient 4 liegt, an dem wiederum eine Referenzanordnung 9 befestigt ist.

Die Referenzanordnungen 7, 8 und 9 können durch das optische Trackingsystem 6 geortet und verfolgt werden, das im vorliegenden Fall eine stereoskopische Kameraanordnung und auch diejenigen elektronischen Bauteile und Programme aufweist, die eine räumliche Ortsbestimmung der Referenzanordnungen 7, 8 und 9 erlauben. Durch die Ortung der Referenzanordnungen 7, 8 und 9 lässt sich nach einer Kalibrierung und/oder Registrierung oder bei vorkalibrierten Systemen auch die räumliche Position des Bauteils bestimmen, gegenüber dem die jeweilige Referenzanordnung unbeweglich angeordnet ist, also beispielsweise die Position der Roboterbasis 2, des Funktionselements 16 oder eines bestimmten Körperteils des Patienten 4, an dem die Referenzanordnung 9 befestigt ist.

Das Trackingsystem 6 bildet damit ein erstes Trackingsystem, das auch als äußeres oder externes Trackingsystem bezeichnet werden könnte. Ein zweites Trackingsystem ist das Trackingsystem, das durch die Gelenksensoren 10', 12' und 14' des Roboters 1 gebildet wird. Einerseits kann also zum Beispiel die Position des Funktionselements 16 über das innere Trackingsystem des Roboters 1 mit den Gelenksensoren 10', 12' und 14' ermittelt werden, andererseits auch durch das äußere Trackingsystem 6 über die Position der Referenzanordnung 7. Es kann also eine redundante Positionsbestimmung durch die beiden Systeme erfolgen, und die entsprechenden Positionsdaten werden an eine Datenverarbeitungs- und/oder Steuereinheit 3 weitergegeben, die typischerweise einen Teil eines medizinischen Navigationssystems bildet.

Es ist noch anzumerken, dass nicht nur die Position des Funktionselements 16 in dieser Weise redundant bestimmt werden kann. Wenn beispielsweise mit dem Funktionselement 16 (das in diesem Fall ein chirurgischer Pointer sein kann) ein Punkt am Patienten angefahren wird, dessen Lage gleichzeitig auch durch die Referenzanordnung 9 dem Trackingsystem 6 mitgeteilt werden kann, kann für diesen Punkt sowohl durch das innere Gelenksensor-Trackingsystems des Roboters 1 als auch durch das äußere Trackingsystem 6 eine Positionsbestimmung erfolgen.

Die Daten aus den beiden Positionsbestimmungen werden in der Steuerungs- bzw. Datenverarbeitungseinheit 3 gesammelt und verarbeitet, und zwar auf eine Weise, die im Weiteren und anhand der Figur 2 erläutert wird. In Figur 1 ist noch zu sehen, dass ein Pfeil von der Einheit 3 zum Roboter 1 (zu dessen Basis 2) führt, und dies soll veranschaulichen, dass eine Robotersteuerung durch die Einheit 3 auf der Basis des erfindungsgemäßen Verfahrens stattfinden kann.

Die vorher angesprochene, erfindungsgemäße Positionsbestimmung, Datenverarbeitung und Robotersteuerung ist in der Figur 2 schematisch als Ablaufdiagramm dargestellt. Der Ablauf beginnt mit dem Sammeln von Trackingdaten aus dem Trackingsystem 6 (äußeres Trackingsystem) sowie Gelenkpositionsdaten, die aus den Gelenksensoren 10', 12' und 14' des Roboters 1 erhalten werden. Diese Positionsdaten werden zusammengeführt (in der Steuerungs- und Datenverarbeitungseinheit 3 aus Figur 1), und es erfolgt eine erfindungsgemäße Prioritätszuordnung auf der Basis von Eingabegrößen, wie sie oben ausführlich beschrieben wurden. In der Figur 2 sind als Eingabegrößen nur exemplarisch externe Parameter und bestimmte Regeln aufgezeigt. Ein Beispiel für einen externen Parameter wäre die Feststellung, dass bestimmte Referenzmarker der Markeranordnung 7 bzw. 9 stark verschmutzt sind und deshalb keine guten Positionsdaten mehr liefern können. Die Priorität würde sich dann zugunsten der Gelenksensor-Positionsdaten verschieben. Ein Beispiel für die Regeln, die der Prioritätszuordnung zugrunde gelegt werden wäre ein Fall, wo nach der letzten Kalibrierung der Gelenksensoren schon eine längere Zeit vergangen ist, so dass deren Positionsmeldungen möglicherweise nicht mehr allzu genau sind. Die Priorität würde sich dann zugunsten der Daten aus dem Trackingsystem verschieben.

Mit der gewählten Prioritätszuordnung kann dann eine Zielposition berechnet werden. Diese Zielposition kann die Position des Körperteils sein, das mit dem Roboter behandelt werden soll, es kann sich aber auch um eine andere im Moment wissenswerte Position handeln, beispielsweise die Position des Funktionselements 16, die sowohl über die Gelenksensoren 10', 12' und 14' als auch über das Trackingsystem 6 mittels der Referenzanordnung 7 bestimmbar ist.

Wenn die Position des zu behandelnden Körperteils die Zielposition ist, kann diese wiederum unter Berücksichtigung weiterer Regeln ermittelt werden, und diese Regeln betreffen beispielsweise Benutzereingaben (Bestätigungen, Korrekturen, Override-Angaben) oder es sind allgemeine Regeln für die Berechnung der Zielposition.

Wenn die Zielposition berechnet worden ist, und zwar unter optimaler Ausnutzung aller zur Verfügung stehenden Informationen, können Anweisungen an den Roboter erzeugt werden, die den Roboter dann zu einer Bewegung veranlassen und ihn in eine neue Position bringen. Diese Position kann eine dem Ziel angenäherte Position oder schon die Zielposition sein, an der der Roboter seine Arbeit verrichtet.

Mit dieser neuen Roboterposition (aus der inneren Positionserfassung) und mit neuen Trackingdaten aus dem äußeren Trackingsystem kann dann der Vorgang kontinuierlich oder intermittierend wiederholt werden, um so über die gesamte Behandlungsdauer eine optimale Navigationsunterstützung bzw. Positionserfassung oder Robotersteuerung bereitstellen zu können.

## Patentansprüche

1. Verfahren zur Bestimmung der Position einer medizintechnischen Einrichtung (9) oder eines Patientenkörperteils, bei dem
- mindestens zwei Positionserfassungen, insbesondere eine erste und eine zweite Positionserfassung, für die Einrichtung (9) oder den Körperteil mit separaten Positionserfassungseinrichtungen (1; 10', 12', 14') durchgeführt werden;
- auf der Basis mindestens einer definierten Eingangsgröße den jeweiligen Positionserfassungen eine bestimmte Priorität zugeordnet wird, welche die Gewichtung der jeweiligen Positionserfassungen bei der Positionsbestimmung definiert; und bei dem
- die Positionsbestimmung unter Berücksichtigung der Gewichtung der jeweiligen Positionserfassungen aus deren Kombination erfolgt.

2. Verfahren nach Anspruch 1, bei dem die jeweiligen Positionserfassungen für einen Zeitpunkt, insbesondere für denselben Zeitpunkt erfolgen.

3. Verfahren nach Anspruch 1 oder Anspruch 2, bei dem die Gewichtung der jeweiligen Positionserfassungen größer als 0% und kleiner als 100% ist.

4. Verfahren nach einem der Ansprüche 1 bis 3, bei dem die Eingangsgröße(n) die Verlässlichkeit und/oder die Genauigkeit der jeweiligen Positionserfassung beschreibt bzw. beschreiben.

5. Verfahren nach einem der Ansprüche 1 bis 4, bei dem die Eingangsgrößen externe Parameter und/oder vordefinierte Prioritätsregeln umfassen.

6. Verfahren nach einem der Ansprüche 1 bis 5, bei dem die Eingangsgröße(n) mindestens eine der folgenden Größen umfasst bzw. umfassen:
- den gemessenen Abstand zwischen einer medizintechnischen Einrichtung (9) und einem Körperteil;
- den Zeitabstand zur letzten Kalibrierung einer jeweiligen Positionserfassungseinrichtung;
- die momentane Erfassbarkeit einer jeweiligen Positionserfassungseinrichtung;
- den Unterschied zwischen den von den jeweiligen Positionserfassungseinrichtungen erfassten Positionen;
- die Berechnung des Verhältnisses der Priorität einer Positionserfassungseinrichtung zu einer anderen Positionserfassungseinrichtung deren Positionserfassung konstant ist;
- einen Durchschnitts- oder Zwischenwert der erfassten Positionen.

7. Verfahren nach einem der Ansprüche 1 bis 6, bei dem bei der Positionsbestimmung, insbesondere auf Abfrage, noch weitere Regelgrößen berücksichtigt werden, insbesondere Benutzereingaben, Benutzerbestätigungen oder Eingriffe in die Positionsbestimmung durch einen Benutzer, insbesondere Benutzer-Override-Eingaben.

8. Verfahren nach einem der Ansprüche 1 bis 7, bei dem die Priorität einer Positionserfassung auf einen bestimmten Zeitabschnitt eingegrenzt wird.

9. Verfahren nach einem der Ansprüche 1 bis 8, bei dem die Prioritätszuordnungen aufgezeichnet werden.

10. Verfahren nach einem der Ansprüche 1 bis 9, bei dem eine der Positionserfassungseinrichtungen ein medizintechnischer Manipulator, insbesondere ein medizintechnischer oder chirurgischer Gelenkroboter (1) ist und eine weitere Positionserfassungseinrichtung ein medizintechnisches optisches oder magnetisches Trackingsystem (6) ist, wobei insbesondere die Eingangsgröße(n) mindestens eine der folgenden Größen umfasst bzw. umfassen:
- die Anzahl der vom Trackingsystem erfassten Referenzmarkierungen, speziell die Anzahl von erfassten Markierungen innerhalb einer Referenzmarkierungsgruppe oder -anordnung (7, 8, 9) bzw. die Qualität der Erfassung;
- die Differenz zwischen Positionsdaten von mindestens zwei redundanten Gelenkpositionssensoren des Roboters (1);
- Kräfte, die von Kraftsensoren am Roboter gemessen werden;
- die Differenz zwischen Positionsdaten vom Gelenkroboter und vom Trackingsystem;
- die Position der Tracking-Referenzmarkierungen im Sichtfeld des Trackingsystems;
- bei durch das Trackingsystem getrackter Roboterbasis der Abstand bzw. die zeitliche Änderung des Abstandes zwischen der Basis und der medizintechnischen Einrichtung / dem Körperteils.

11. Verfahren zum Steuern eines medizintechnischen Manipulators, insbesondere eines medizintechnischen Roboters (1), bei dem der Manipulator mit Hilfe von Positionsinformationen gesteuert wird, die mit einem Positionsbestimmungsverfahren nach einem der Ansprüche 1 bis 10 erhalten wurden.

12. Verfahren nach Anspruch 11, bei dem Positionseingabedaten für die Manipulator- bzw. Robotersteuerung geändert werden, wenn eine Änderung der Priorität erfolgt.

13. Verfahren nach Anspruch 11 oder 12, bei dem Positionseingabedaten für die Manipulator- bzw. Robotersteuerung durch einen Benutzereingriff geändert werden.

14. Verfahren nach einem der Ansprüche 11 bis 13, bei dem Positionseingabedaten für die Manipulator- bzw. Robotersteuerung nur geändert werden, wenn der Unterschied zwischen verschiedenen Positionserfassungsdaten einen Schwellwert erreicht oder überschreitet.

15. Verfahren nach einem der Ansprüche 11 bis 14, bei dem mittels einer Signalausgabe die Gewichtung der Positionserfassungen angezeigt wird.

16. Programm, das, wenn es auf einem Computer läuft oder in einem Computer geladen ist, den Computer veranlasst, ein Verfahren gemäß einem der Ansprüche 1 bis 15 durchzuführen.

17. Computerprogramm-Speichermedium, das ein Programm nach Anspruch 16 aufweist.

18. Vorrichtung zur Bestimmung der Position einer medizintechnischen Einrichtung (9) oder eines Patientenkörperteils, mit mindestens zwei separaten Positionserfassungseinrichtungen (1; 10', 12', 14') zur Positionserfassung für die Einrichtung (9) oder den Körperteil, **gekennzeichnet durch** einer Steuerungs- und Datenverarbeitungseinheit, die eine Speicher- oder Eingabeeinheit für mindestens eine definierte Eingangsgröße aufweist, und welche auf der Basis der mindestens einen definierten Eingangsgröße den jeweiligen Positionserfassungen eine bestimmte Priorität zuordnet, welche die Gewichtung der jeweiligen Positionserfassungen bei der Positionsbestimmung definiert, und die Position der Einrichtung (9) oder des Körperteils unter Berücksichtigung der Gewichtung der jeweiligen Positionserfassungen aus deren Kombination bestimmt.

19. Vorrichtung zum Steuern eines medizintechnischen Manipulators, insbesondere eines medizintechnischen Roboters (1), wobei der Manipulator mit Hilfe von Positionsinformationen gesteuert wird, die mit einer Positionsbestimmungvorrichtung nach dem Anspruch 18 erhalten wurden.
